# EUROPEAN PATENT APPLICATION

(11) **EP 4 239 582 A1**
(43) Date of publication of application: **06.09.2023**
(21) Application number: 23159713.9
(22) Date of filing: 02.03.2023
(51) Int. Cl.: G06T 11/00, A61B 5/327

(54) **MEDICAL IMAGE DIAGNOSIS APPARATUS, IMAGE RECONSTRUCTION METHOD, AND COMPUTER-READABLE STORAGE MEDIUM STORING THEREIN IMAGE RECONSTRUCTION PROGRAM**

(30) Priority: 04.03.2022 JP 2022033729
(71) Applicant: Canon Medical Systems Corporation, Tochigi 324-0036 (JP)
(72) Inventor: TESHIGAWARA, Manabu, Otawara-shi, 324-0036 (JP)
(74) Representative: Marks & Clerk LLP

(57) **Abstract**

A medical image diagnosis apparatus (1) includes a data obtaining unit (730), a pulse wave information obtaining unit (735), and a reconstructing unit (731). The data obtaining unit (730) obtains scan data generated by scanning an examined subject. The pulse wave information obtaining unit (735) obtains pulse wave information of the examined subject, along with the scan. The reconstructing unit (731) performs image reconstruction corresponding to electrocardiogram synchronization of the examined subject, by using the pulse wave information and the scan data.

## Description

### FIELD

Embodiments described herein relate generally to a medical image diagnosis apparatus, an image reconstruction method, and a computer-readable storage medium storing therein an image reconstruction program.

### BACKGROUND

Conventionally, a dedicated monitor is used for measuring cardiac voltage displacement data of an examined subject during electrocardiogram-synchronized imaging, such as X-ray Computed Tomography (hereinafter, "X-ray CT"), Positron Emission Tomography (hereinafter, "PET"), Single Photon Emission Computed Tomography (hereinafter, "SPECT"), or the like. The cardiac voltage displacement data is saved together with time-series acquisition data obtained by scanning the examined subject. A specific phase designated from among pulsation phases of the examined subject is to be used for image reconstruction synchronized with an electrocardiogram.

However, medical examinations involving electrocardiogram-synchronized imaging require to directly attach electrocardiographic ports to the body surface of the examined subject and to arrange cables extending from the electrocardiographic ports to be routed from the examined subject placed in a scanner to a dedicated monitor. When the cables are not properly routed around, the electrocardiographic ports may come off the patient at the time of moving a tabletop on which the patient is placed, which may hinder the medical examination. Further, because the conductive wires in the cables are made of a scattering material for X-rays and gamma rays, the wires can be a cause of image quality degradation.

To cope with the circumstances described above, there is a demand for a method for acquiring a signal from the examined subject, the signal being used for dividing phases of pulsation with respect to scan data, while eliminating the need to route the cables around and avoiding the scattering of X-rays and gamma rays. For example, a method is known by which a heartbeat waveform substituting for an electrocardiogram is contactlessly estimated, for example, by detecting a pulse wave with an optical camera and converting the waveform. However, this method is used for the purpose of finding out an activity state such as exercise or driving or arrhythmia, without using an electrocardiograph monitor. Thus, this method is not required to be temporally consistent with the physically-acquired scan data, unlike the mode of use for realizing the electrocardiogram-synchronized image reconstruction.

### SUMMARY OF THE INVENTION

A medical image diagnosis apparatus including: a data obtaining unit configured to obtain scan data generated by scanning an examined subject; a pulse wave information obtaining unit configured to obtain pulse wave information of the examined subject, along with the scan; and a reconstructing unit configured to perform image reconstruction corresponding to electrocardiogram synchronization of the examined subject, by using the pulse wave information and the scan data.

The medical image diagnosis apparatus may further include an estimating unit configured to estimate a timing shift between the pulse wave information and an electrocardiogram waveform of the examined subject. The reconstructing unit may be configured to perform the image reconstruction corresponding to the electrocardiogram synchronization, by further using the timing shift.

The estimating unit may be configured to estimate the timing shift, through a calculation based on a correspondence table indicating timing shifts corresponding to physique information of the examined subject.

The estimating unit may be configured to estimate the timing shift, by inputting the pulse wave information to a trained model trained to output the timing shift in response to receiving an input of the pulse wave information.

The estimating unit may be configured to estimate the timing shift, by inputting the pulse wave information and physique information of the examined subject to a trained model trained to output the timing shift, in response to receiving an input of the pulse wave information and geometric information related to the examined subject.

The medical image diagnosis apparatus may further include a phase dividing unit configured to divide an interval between two pulse wave peaks adjacent to each other in the pulse wave information, into a plurality of phases. The reconstructing unit may be configured to generate a plurality of reconstruction images corresponding to the plurality of phases, on a basis of the scan data included in each of the plurality of phases. The estimating unit may be configured to generate a time-volume curve related to a heart of the examined subject on a basis of the plurality of reconstruction images; and to estimate the timing shift on a basis of the time-volume curve.

The pulse wave information obtaining unit may be configured either to contactlessly obtain the pulse wave information from the examined subject or to obtain the pulse wave information from a pulse wave meter provided in an end part of the examined subject within a non-imaged range during the scan.

The medical image diagnosis apparatus may further include a phase dividing unit configured to divide an interval between two pulse wave peaks adjacent to each other in the pulse wave information, into a plurality of phases. The reconstructing unit may be configured to shift an initial phase corresponding to one of the peaks among the plurality of phases, multiple times toward past at predetermined time intervals; and to generate a plurality of reconstruction images corresponding to how many times the shift was made, on a basis of the scan data included in two or more of the initial phases that were shifted.

The reconstructing unit may be configured to identify a sharpest image among the plurality of reconstruction images as a reconstruction image corresponding to the electrocardiogram synchronization.

The medical image diagnosis apparatus may further include a phase dividing unit configured to divide an interval between two pulse wave peaks adjacent to each other in the pulse wave information, into a plurality of phases. The reconstructing unit may be configured to shift an initial phase corresponding to one of the peaks among the plurality of phases, multiple times toward past at predetermined time intervals; to generate a plurality of reconstruction images corresponding to how many times the shift was made, on a basis of the scan data included in two or more of the initial phases that were shifted; and to identify a sharpest image among the plurality of reconstruction images, by inputting the plurality of reconstruction images to a trained model trained to identify the sharpest image in response to receiving an input of the plurality of reconstruction images.

An image reconstruction method includes obtaining scan data by scanning an examined subject; obtaining pulse wave information of the examined subject, along with the scan; and performing image reconstruction corresponding to electrocardiogram synchronization of the examined subject, by using the pulse wave information and the scan data.

A computer-readable storage medium stores therein an image reconstruction program that causes a computer to realize: obtaining scan data by scanning an examined subject; obtaining pulse wave information of the examined subject, along with the scan; and performing image reconstruction corresponding to electrocardiogram synchronization of the examined subject, by using the pulse wave information and the scan data.

### BRIEF DESCRIPTION OF THE DRAWINGS

FIG. 1 is a diagram illustrating a configuration of a Positron Emission Tomography Computed Tomography (PET-CT) apparatus according to a first embodiment;
FIG. 2 is a drawing according to the first embodiment illustrating an example of a difference in phase (a phase difference) between an electrocardiogram waveform and a pulse wave;
FIG. 3 is a drawing according to the first embodiment illustrating an example of an outline of an electrocardiogram-synchronized reconstruction process;
FIG. 4 is a flowchart according to the first embodiment illustrating an example of a procedure in the electrocardiogram-synchronized reconstruction process;
FIG. 5 is a diagram according to a modification example of the first embodiment illustrating an example of a functional configuration of processing circuitry;
FIG. 6 is a chart illustrating an example of a time-volume curve according to the modification example of the first embodiment; and
FIG. 7 is a drawing according to a second embodiment illustrating phase division in pulse wave information (a pulse waveform) and an example of a phases of a reconstruction image corresponding to electrocardiogram synchronization based on shifting of an initial phase in the pulse waveform.

### DETAILED DESCRIPTION

A medical image diagnosis apparatus according to an embodiment includes processing circuitry. The processing circuitry is configured: to obtain scan data generated by scanning an examined subject; to obtain pulse wave information of the examined subject, along with the scan; and to perform image reconstruction corresponding to electrocardiogram synchronization of the examined subject, by using the pulse wave information and the scan data.

Exemplary embodiments of a medical image diagnosis apparatus, an image reconstruction method, and an image reconstruction program will be explained in detail below, with reference to the accompanying drawings. In the following embodiments, some of the elements referred to by using the same reference characters are assumed to perform the same operations, and duplicate explanations thereof will be omitted as appropriate. Further, possible embodiments of the medical image diagnosis apparatus, the image reconstruction method, and the image reconstruction program of the present disclosure are not limited to the embodiments described below.

A medical image diagnosis apparatus according to an embodiment of the present disclosure has an imaging mechanism used for the purpose of obtaining a medical image related to electrocardiogram synchronization (hereinafter, "EKG synchronization") from an examined subject (hereinafter, "patient"). For example, the medical image diagnosis apparatus includes an imaging mechanism configured to perform a Positron Emission Tomography (PET) imaging process. Examples of the medical image diagnosis apparatus include: a PET apparatus having only a PET imaging function; a Positron Emission Tomography Computed Tomography (PET-CT) apparatus including a PET imaging mechanism and an X-ray Computed Tomography (CT) imaging mechanism; and a Positron Emission Tomography Magnetic Resonance (PET-MR) apparatus including a PET imaging mechanism and a Magnetic Resonance (MR) imaging mechanism. Further, the medical image diagnosis apparatus according to the present embodiment may include an imaging mechanism configured to perform a Single Photon Emission Computed Tomography (SPECT) imaging process. Examples of this type of medical image diagnosis apparatus include: a SPECT apparatus including only a SPECT imaging mechanism; a SPECT-CT apparatus including a SPECT imaging mechanism and a CT imaging mechanism; and a SPECT-MR apparatus including a SPECT imaging mechanism and an MR imaging mechanism.

In another example, the medical image diagnosis apparatus according to the present embodiment may include only an MR imaging mechanism. Examples of this type of medical image diagnosis apparatus include a Magnetic Resonance Imaging (MRI) apparatus including only an MR imaging mechanism. In yet another example, the medical image diagnosis apparatus according to the present embodiment may include only a CT imaging mechanism. Examples of this type of medical image diagnosis apparatus include an X-ray CT apparatus including only a CT imaging mechanism. Although any of these types of apparatuses is applicable, to explain a specific example, it will be assumed that the medical image diagnosis apparatus according to the present embodiment is a PET-CT apparatus.

### First Embodiment

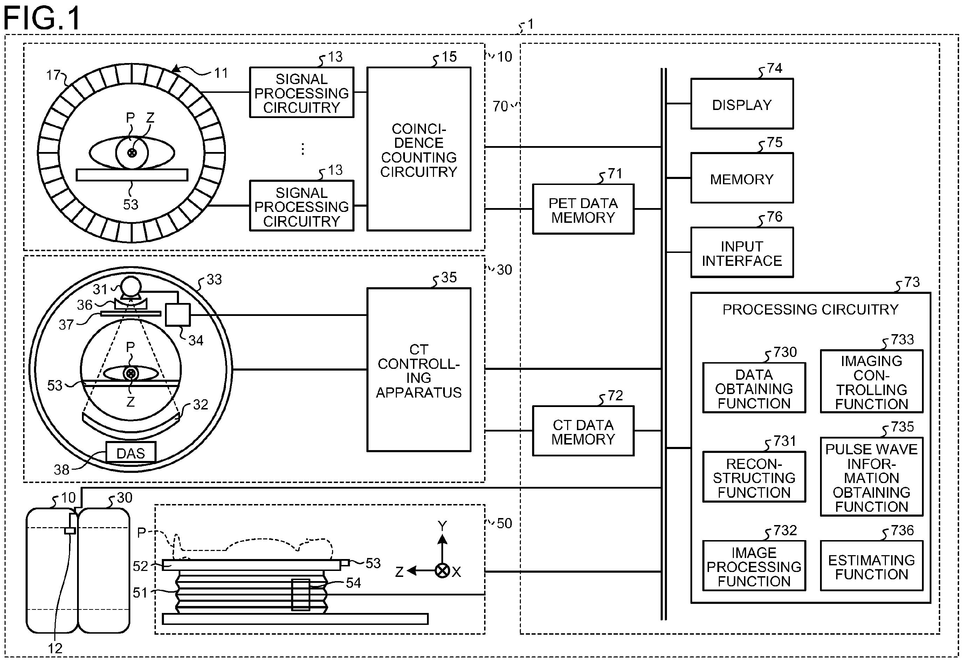

FIG. 1 is a diagram illustrating a configuration of a PET-CT apparatus 1 according to a first embodiment. As illustrated in FIG. 1, the PET-CT apparatus 1 includes a PET gantry 10, a CT gantry 30, a table 50, and a console 70. Typically, the PET gantry 10, the CT gantry 30, and the table 50 are installed in mutually the same examination room. The console 70 is installed in a control room adjacent to the examination room. The PET gantry 10 is an imaging apparatus for performing a PET imaging process (a PET scan) on the patient P. The CT gantry 30 is an imaging apparatus for performing an X-ray CT imaging process (a CT scan) on the patient P. The table 50 is configured to movably support a tabletop 53 on which the patient P to be imaged is placed. The console 70 is a computer configured to control the PET gantry 10, the CT gantry 30, the table 50, and the like.

As illustrated in FIG. 1, the PET gantry 10 includes, for example, a detector ring 11, signal processing circuitry 13, and coincidence counting circuitry 15. In this situation, the PET gantry 10 and the CT gantry 30 may be housed in mutually the same casing. Further, the PET gantry 10 is provided with a camera 12 capable of imaging the face of the patient P or a neck part (the body surface in the vicinity of the carotid artery) or the like of the patient P. As for the installation position, the camera may be provided in any position as long as it is possible to image the face of the patient P during the PET imaging process performed for the patient P. The camera 12 may be an optical camera, for example; however, the camera 12 does not necessarily have to be an optical camera and may be any type of camera as long as it is possible to image changes in the face or displacements of the carotid artery of the patient. Further, the camera 12 may be provided for the CT gantry 30. In that situation, as for the installation position, the camera may be provided in any position as long as it is possible to image the face of the patient P during the CT imaging process performed for the patient P. When the camera is not provided on a casing, a wearable device capable of measuring a pulse wave such as a pulse wave meter (e.g., a pulse oximeter) is attached to an end part of the patient P within a non-imaged range (a non-Field of View (non-FOV) range), during scans such as the PET imaging process and the CT imaging process. Video data taken by the camera 12 or data output from the pulse wave meter is output to the console 70 in a wired or wireless manner.

The detector ring 11 includes a plurality of gamma ray detectors 17 arranged in a circle formation around a central axis Z. An opening part of the detector ring 11 is set with a Field Of View (FOV). The position of the patient P is determined in such a manner that an imaged site of the patient P is included in the field of view. A drug labeled with positron emitting nuclei is administered for the patient P. Positrons emitted from the positron emitting nuclei annihilate with electrons in the surroundings. As a result of the annihilation, a pair of annihilation gamma rays is generated. The gamma ray detectors 17 are configured to detect the annihilation gamma rays emitted from the inside of the body of the patient P. The gamma ray detectors 17 are configured to generate electrical signals corresponding to the amounts of light of the detected annihilation gamma rays. For example, the gamma ray detectors 17 include a plurality of scintillators and a plurality of photomultiplier tubes. The scintillators are configured to receive the annihilation gamma rays occurring from a radioactive isotope inside the patient P and to generate scintillation light. The photomultiplier tubes are configured to generate the electrical signals corresponding to the amounts of light of the scintillation light. The generated electrical signals are supplied to the signal processing circuitry 13.

The signal processing circuitry 13 are configured to generate single event data on the basis of the electrical signals output from the gamma ray detectors 17. More specifically, the signal processing circuitry 13 are configured to perform, for example, a detection time measuring process, a position calculating process, and an energy calculating process on the electrical signals. The signal processing circuitry 13 are each realized by using an Application Specific Integrated Circuit (ASIC), a Field Programmable Gate Array (FPGA), or other types of devices such as a Complex Programmable Logic Device (CPLD) or a Simple Programmable Logic Device (SPLD), configured to be able to perform the detection time measuring process, the position calculating process, and the energy calculating process.

In the detection time measuring process, each of the signal processing circuitry 13 is configured to measure a gamma ray detection time of a corresponding one of the gamma ray detectors 17. More specifically, each of the signal processing circuitry 13 is configured to monitor crest values of the electrical signal from a corresponding one of the gamma ray detectors 17 and to measure the time at which a crest value exceeds a threshold value set in advance as the detection time. In other words, by detecting that the crest value has exceeded the threshold value, each of the signal processing circuitry 13 is configured to electrically detect the annihilation gamma rays. In the position calculating process, each of the signal processing circuitry 13 is configured to calculate an incident position of the annihilation gamma rays on the basis of the electrical signal from a corresponding one of the gamma ray detectors 17. The incident position of the annihilation gamma rays corresponds to position coordinates of a scintillator to which the annihilation gamma rays have become incident. In the energy calculating process, each of the signal processing circuitry 13 is configured to calculate an energy value of the detected annihilation gamma rays, on the basis of the electrical signal from a corresponding one of the gamma ray detectors 17.

Data of the detection time related to the single event is brought into association with data of the position coordinates and with data of the energy value. A set made up of the data of the energy value, the data of the position coordinates, and the data of the detection time related to the single event will be referred to as single event data. The single event data is sequentially generated every time annihilation gamma rays are detected. The generated single event data is supplied to the coincidence counting circuitry 15.

The coincidence counting circuitry 15 is configured to perform a coincidence counting process on the single event data from the signal processing circuitry 13. As for hardware resources thereof, the coincidence counting circuitry 15 is realized by using an ASIC, an FPGA, a CPLD, or an SPLD configured to be able to perform the coincidence counting process. In the coincidence counting process, the coincidence counting circuitry 15 is configured to repeatedly identify, from among pieces of single event data repeatedly supplied, a piece of single event data related to two single events contained in a predetermined time frame. It is speculated that the pair of single events occur from annihilation gamma rays generated at mutually the same annihilation points. The pairs of single events are collectively referred to as coincidence events. A line connecting together a pair of gamma ray detectors 17 (or scintillators, more specifically) that have detected the annihilation gamma rays is called a Line Of Response (LOR). The event data related to the pair of events connected by the LOR is called coincidence event data. The coincidence event data and the single event data are transferred to the console 70. In the following sections, when the coincidence event data and the single event data are not particularly distinguished from each other, the two types of data will collectively be referred to as PET event data.

Further, although the signal processing circuitry 13 and the coincidence counting circuitry 15 are described above as being included in the PET gantry 10, the present embodiment is not limited to this configuration. For example, the coincidence counting circuitry 15 or both the signal processing circuitry 13 and the coincidence counting circuitry 15 may be included in another apparatus different from the PET gantry 10. Further, one coincidence counting circuitry 15 may be provided for the plurality of signal processing circuitry 13 included in the PET gantry 10. Alternatively, the plurality of signal processing circuitry 13 included in the PET gantry 10 may be separated into a plurality of groups, so that one coincidence counting circuitry 15 is provided for each of the groups.

As illustrated in FIG. 1, the CT gantry 30 includes an X-ray tube 31, an X-ray detector 32, a rotating frame 33, an X-ray high-voltage apparatus 34, a CT controlling apparatus 35, a wedge 36, a collimator 37, and a Data Acquisition System (DAS) 38.

The X-ray tube 31 is configured to generate X-rays. More specifically, the X-ray tube 31 includes a vacuum tube holding a negative pole configured to generate thermo electrons and a positive pole configured to receive the thermo electrons flying from the negative pole and to generate the X-rays. The X-ray tube 31 is connected, via a high-voltage cable, to the X-ray high-voltage apparatus 34. X-ray tube voltage is applied to between the negative pole and the positive pole by the X-ray high-voltage apparatus 34. As a result of the application of the X-ray tube voltage, the thermo electrons fly from the negative pole toward the positive pole. As a result of the thermo electrons flying from the negative pole toward the positive pole, an X-ray tube current flows. As a result of the application of the high voltage and a supply of a filament current from the X-ray high-voltage apparatus 34, the thermo electrons fly from the negative pole toward the positive pole, so that the thermo electrons collide with the positive pole. As a result, the X-rays are generated.

The X-ray detector 32 is configured to detect X-rays that were generated from the X-ray tube 31 and have passed through the patient P. The X-ray detector 32 is configured to output an electrical signal corresponding to a detected radiation amount of the X-rays to the DAS 38. The X-ray detector 32 has a structure in which a plurality of rows of X-ray detecting elements are arranged in a slice direction (which may be referred to as a row direction), while each of the rows includes a plurality of X-ray detecting elements arranged in a channel direction. For example, the X-ray detector 32 is a detector of an indirect conversion type including a grid, a scintillator array, and an optical sensor array. The scintillator array includes a plurality of scintillators. Each of the scintillators is configured to output light in a light amount corresponding to the amount of X-rays becoming incident thereto. The grid is provided on the X-ray incident surface side of the scintillator array. The grid has an X-ray blocking plate configured to absorb scattered X-rays. The optical sensor array is configured to convert the light output from the scintillators into the electrical signal corresponding to the light amount of the light. As the optical sensor, it is possible to use a photodiode or a photomultiplier tube, for example. Alternatively, the X-ray detector 32 may be realized by using a detector (a semiconductor detector) of a direct conversion type including a semiconductor element configured to convert the incident X-rays into an electrical signal.

The rotating frame 33 is an annular frame configured to support the X-ray tube 31 and the X-ray detector 32 so as to be rotatable on a rotation axis Z. More specifically, the rotating frame 33 is configured to support the X-ray tube 31 and the X-ray detector 32 so as to oppose each other. The rotating frame 33 is supported by a fixed frame (not illustrated) so as to be rotatable on the rotation axis Z. Under control of the CT controlling apparatus 35, the rotating frame 33 rotates on the rotation axis Z. As a result, the X-ray tube 31 and the X-ray detector 32 rotate on the rotation axis Z. The rotating frame 33 is configured to rotate with constant angular velocity on the rotation axis Z, while receiving motive power from a driving mechanism of the CT controlling apparatus 35. An opening part of the rotating frame 33 is set with a Field Of View (FOV).

In the present embodiment, the rotation axis of the rotating frame 33 in a non-tilted state or the longitudinal direction of the tabletop 53 of the table 50 is defined as a Z-axis direction; an axial direction orthogonal to the Z-axis direction and parallel to the floor surface is defined as an X-axis direction; and an axial direction orthogonal to the Z direction and parallel to the floor surface is defined as a Y-axis direction.

The X-ray high-voltage apparatus 34 includes electric circuitry such as a transformer and a rectifier. Further, the X-ray high-voltage apparatus 34 includes: a high-voltage generating apparatus configured to generate the high voltage to be applied to the X-ray tube 31 and the filament current to be supplied to the X-ray tube 31; and an X-ray controlling apparatus configured to control output voltage corresponding to the X-rays to be emitted by the X-ray tube 31. The high-voltage generating apparatus may be of a transformer type or an inverter type. The X-ray high-voltage apparatus 34 may be provided on the rotating frame 33 in the CT gantry 30 or may be provided on a fixed frame (not illustrated) in the CT gantry 30.

The wedge 36 is configured to adjust the radiation amount of the X-rays to be emitted onto the patient P. More specifically, the wedge 36 is configured to attenuate the X-rays so that the radiation amount of the X-rays emitted from the X-ray tube 31 onto the patient P has a predetermined distribution. For example, as the wedge 36, it is possible to use a metal plate of aluminum or the like, such as a wedge filter or a bow-tie filter.

The collimator 37 is configured to limit an emission range of the X-rays that have passed through the wedge 36. The collimator 37 is configured to slidably support a plurality of lead plates that block the X-rays and to adjust the shapes of slits formed by the plurality of lead plates.

The Data Acquisition System (DAS) 38 is configured to read, from the X-ray detector 32, the electrical signal corresponding to the radiation amount of the X-rays detected by the X-ray detector 32. The DAS 38 is configured to amplify the read electrical signal with a variable amplification rate. Subsequently, by integrating the amplified electrical signal over a view time period, the DAS 38 is configured to acquire CT raw data having a digital value corresponding to the radiation amount of the X-rays in the view time period. For example, the DAS 38 is realized by using an ASIC including a circuit element capable of generating the CT raw data. The CT raw data is transferred to the console 70 via a contactless data transfer apparatus or the like.

By employing an imaging controlling function 733 of processing circuitry 73 included in the console 70, the CT controlling apparatus 35 is configured to control the X-ray high-voltage apparatus 34, the DAS 38, and the like, to execute the X-ray CT imaging process. The CT controlling apparatus 35 includes processing circuitry including a Central Processing Unit (CPU) or the like and a driving mechanism configured with a motor and an actuator, or the like. As hardware resources thereof, the processing circuitry includes a processor such as the CPU or a Micro Processing Unit (MPU) and memory elements such as a Read Only Memory (ROM), a Random Access Memory (RAM), and/or the like. Alternatively, the CT controlling apparatus 35 may be realized by using an ASIC, an FPGA, a CPLD, an SPLD, or the like.

Examples of the CT gantry 30 include various types such as: a Rotate/Rotate Type (a third-generation CT) in which an X-ray generating unit and an X-ray detecting unit integrally rotate around the patient; and a Stationary/Rotate Type (a fourth-generation CT) in which only an X-ray generating unit rotates around the patient, while a large number of X-ray detecting elements arrayed in a ring formation are fixed. It is possible to apply any of these types to each of the embodiments.

As illustrated in FIG. 1, the table 50 is configured to have the patient P to be scanned placed thereon and to move the placed patient. The table 50 is shared by the PET gantry 10 and the CT gantry 30.

The table 50 includes a base 51, a supporting frame 52, the tabletop 53, and a table driving apparatus 54. The base 51 is installed on the floor surface. The base 51 is a casing configured to support the supporting frame 52 so as to be movable in a direction (the Y-axis direction) perpendicular to the floor surface. The supporting frame 52 is a frame provided above the base 51. The supporting frame 52 is configured to support the tabletop 53 so as to be slidable along the central axis Z. The tabletop 53 is a flexible board on which the patient P is placed.

The table driving apparatus 54 is housed in the casing of the table 50. The table driving apparatus 54 is a motor or actuator configured to generate the motive power for moving the supporting frame 52 and the tabletop 53 over which the patient P is placed. The table driving apparatus 54 is configured to operate according to control exercised by the console 70 and the like.

The PET gantry 10 and the CT gantry 30 are positioned in such a manner that the central axis Z of the opening of the PET gantry 10 substantially coincides with the central axis Z of the opening of the CT gantry 30. The table 50 is positioned in such a manner that the long axis of the tabletop 53 extends parallel to the central axes Z of the openings of the PET gantry 10 and the CT gantry 30. The CT gantry 30 and the PET gantry 10 are arranged so that, for example, the CT gantry 30 is positioned closer to the table 50 than the PET gantry 10 is.

As illustrated in FIG. 1, the console 70 includes a PET data memory 71, a CT data memory 72, the processing circuitry 73, a display 74, a memory 75, and an input interface 76. For example, data communication among the PET data memory 71, the CT data memory 72, the processing circuitry 73, the display 74, the memory 75, and the input interface 76 is performed via a bus.

The PET data memory 71 is a storage device configured to store therein the single event data and the coincidence event data transferred thereto from the PET gantry 10. The PET data memory 71 is a storage device such as a Hard Disk Drive (HDD), a Solid State Drive (SSD), or an integrated circuit storage device.

The CT data memory 72 is a storage device configured to store therein the CT raw data transferred thereto from the CT gantry 30. The CT data memory 72 is a storage device such as an HDD, an SSD, or an integrated circuit storage device.

As hardware resources thereof the processing circuitry 73 includes a processor such as a CPU, an MPU, or a Graphics Processing Unit (GPU) and memory elements such as a ROM, a RAM, and/or the like. The processing circuitry 73 is configured to realize a data obtaining function 730, a reconstructing function 731, an image processing function 732, the imaging controlling function 733, a pulse wave information obtaining function 735, and an estimating function 736, by executing various types of programs read from one or more of the memory elements. In other words, the processing circuitry 73 corresponds to a processor configured to realize the functions corresponding to the programs by reading and executing the programs from the memory elements. That is to say, the processing circuitry 73 that has read the programs has the functions corresponding to the read programs. In this situation, the data obtaining function 730, the reconstructing function 731, the image processing function 732, the imaging controlling function 733, the pulse wave information obtaining function 735, and the estimating function 736 may be implemented by the processing circuitry 73 realized with one circuit board or may be implemented by the processing circuitry 73 realized with a plurality of circuit boards in a distributed manner. The processing circuitry 73 realizing the data obtaining function 730, the reconstructing function 731, the image processing function 732, the imaging controlling function 733, the pulse wave information obtaining function 735, and the estimating function 736 corresponds to a data obtaining unit, a reconstructing unit, an image processing unit, an imaging controlling unit, a pulse wave information obtaining unit, and an estimating unit, respectively.

By employing the data obtaining function 730, the processing circuitry 73 is configured to obtain scan data by scanning the patient P. The scan data may be, for example, the coincidence event data and/or the CT raw data. For example, when the PET imaging process is performed for the patient P, the data obtaining function 730 obtains the coincidence event data from the PET data memory 71. In contrast, when the CT imaging process is performed for the patient P, the data obtaining function 730 obtains the CT raw data from the CT data memory 72. Alternatively, the functions realized by the data obtaining function 730 may be realized by the reconstructing function 731 or the imaging controlling function 733, for example. In yet another example, the data obtaining function 730 may be realized by the PET gantry 10 and/or the CT gantry 30. In that situation, the data obtaining function 730 is configured to obtain the coincidence event data from the PET imaging process performed on the patient P and to obtain the CT raw data from the CT imaging process performed on the patient P.

By employing the reconstructing function 731, the processing circuitry 73 is configured to reconstruct a PET image indicating a distribution of positron emitting nuclei administered for the patient P, on the basis of the coincidence event data obtained by the data obtaining function 730. Further, the processing circuitry 73 is configured to reconstruct a CT image expressing a spatial distribution of CT values related to the patient P, on the basis of the CT raw data obtained by the data obtaining function 730. As an image reconstruction algorithm, an existing image reconstruction algorithm based on Filtered Backprojection (FBP) or a successive approximation reconstruction method may be used. Further, the processing circuitry 73 is also capable of generating a position determining image related to PET on the basis of the PET event data and generating a position determining image related to CT on the basis of the CT raw data. Further, by employing the reconstructing function 731, the processing circuitry 73 is configured to perform image reconstruction (hereinafter, "electrocardiogram-synchronized (EKG-synchronized) image reconstruction") corresponding to EKG synchronization of the patient P, by using pulse wave information and the scan data of the patient P. The EKG-synchronized image reconstruction corresponds to reconstructing a medical image corresponding to EKG synchronization, by using scan data included in a phase width related to R-wave times, among a plurality of divided phases obtained by performing phase division on the pulse wave information, for example. More specifically, the reconstructing function 731 is configured to perform the EKG-synchronized image reconstruction by further using a timing shift estimated by the estimating function 736, in addition to the pulse wave information and the scan data of the patient P. Processes performed in the EKG-synchronized image reconstruction (hereinafter, "EKG-synchronized reconstruction process") will be explained later.

By employing the image processing function 732, the processing circuitry 73 is configured to perform various types of image processing processes on the PET image and the CT image reconstructed by the reconstructing function 731. For example, the processing circuitry 73 is configured to generate display images by performing a three-dimensional image processing process, such as volume rendering, surface volume rendering, a pixel value projection process, a Multi-Planar Reconstruction (MPR) process, or a Curved MPR (CPR) process on the PET image and the CT image. In addition, the image processing function 732 may perform a cardiac function analysis on the basis of a PET image related to the heart of the patient P, for example.

By employing the imaging controlling function 733, the processing circuitry 73 is configured to control the PET gantry 10 and the table 50 in a synchronized manner, so as to perform the PET imaging process. The PET imaging process in the present embodiment is assumed to be an intermittent moving scan (a step-and-shoot method) by which PET event data is acquired from each acquisition area while the tabletop 53 is intermittently moved. Further, The processing circuitry 73 is configured to control the CT gantry 30 and the table 50 in a synchronized manner, so as to perform the CT imaging process. When the PET imaging process and the CT imaging process are successively performed, the imaging controlling function 733 is configured to control the PET gantry 10, the CT gantry 30, and the table 50 in a synchronized manner. Further, the processing circuitry 73 is also capable of performing a position determining scan employing the PET gantry 10 (hereinafter, "PET position determining scan") and a position determining scan employing the CT gantry 30 (hereinafter, "CT position determining scan"). To perform the PET position determining scan, the processing circuitry 73 is configured to control the PET gantry 10 and the table 50 in a synchronized manner. To perform the CT position determining scan, the processing circuitry 73 is configured to control the CT gantry 30 and the table 50 in a synchronized manner.

By employing the pulse wave information obtaining function 735, the processing circuitry 73 is configured to obtain the pulse wave information of the patient P, along with the scan. For example, the pulse wave information obtaining function 735 is configured either to obtain the pulse wave information from the patient P contactlessly (e.g., by using the camera 12) or to obtain the pulse wave information from a pulse wave meter (e.g., a pulse oximeter) provided in the end part of the patient within the non-imaged range during the scan.

More specifically, when the face of the patient P is imaged by the camera 12, the pulse wave information obtaining function 735 is configured to obtain the pulse wave information of the patient P, on the basis of the video data output from the camera 12. More specifically, the pulse wave information obtaining function 735 is configured to obtain the pulse wave information, according to small changes in face complexion on the face of the patient P rendered in the video data output from the camera 12. Because it is possible to apply any of existing methods, as appropriate, to the process of obtaining the pulse wave information on the basis of the small changes in the face complexion, explanations thereof will be omitted. Further, when a wearable device such as the pulse oximeter to measure the pulse wave is attached to the patient P, the pulse wave information obtaining function 735 is configured to obtain the pulse wave information of the patient, by receiving data of the pulse wave output from the wearable device. The pulse wave information obtaining function 735 is configured to store, into the memory 75, the pulse wave information and the scan data so as to be kept in association with each other on the basis of the obtainment time of the pulse wave and the obtainment time of the scan data.

By employing the estimating function 736, the processing circuitry 73 is configured to estimate a difference in phase (hereinafter, " timing shift") between the pulse wave information and an electrocardiogram waveform (hereinafter, "EKG waveform") of the patient P. For reconstructing a phase image by using the EKG waveform, a phase starting at an R-wave peak is usually used. For this reason, in order to correctly divide the scan data in the time series at the R-wave starting point by referencing the pulse wave, it is necessary to correct the difference in phase between the pulse wave and the EKG waveform with respect to the pulse wave. The difference in phase includes: a delay caused by the distance between the heart and the site in which the pulse wave is detected; and a difference in phase caused by deformation of the pulse wave due to propagation of the blood flow. In other words, it is not that peaks of the pulse wave directly correspond to peaks of the R-wave.

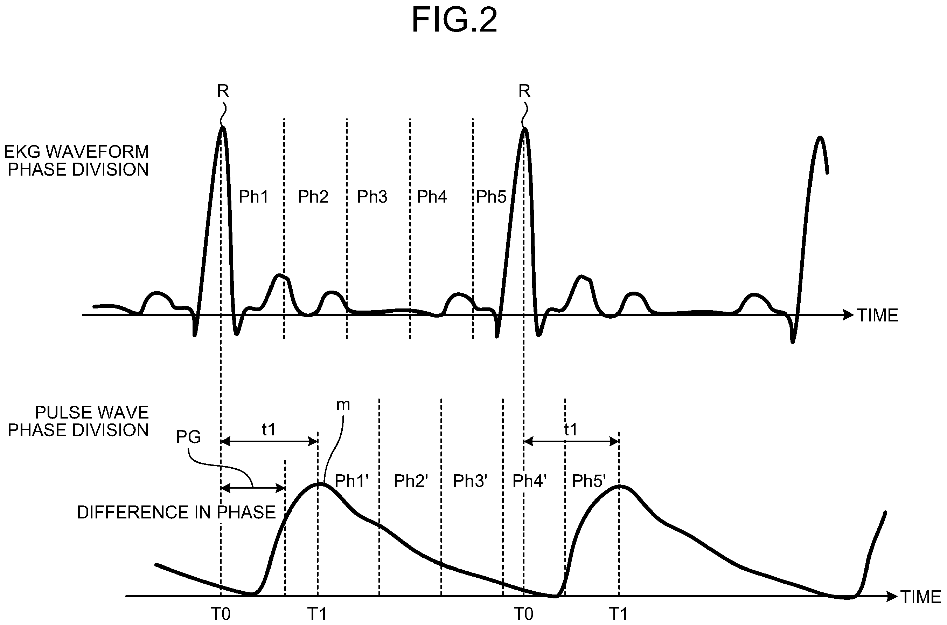

FIG. 2 is a drawing illustrating an example of the difference in phase (a phase difference) between an EKG waveform and a pulse wave. As illustrated in FIG. 2, phases of the pulse wave are different from phases of the EKG waveform. Further, because there may be differences in the peak positions due to deformation of the waveform during the propagation process of the pulse wave, the peaks of the pulse wave do not directly correspond to the peaks of the R-wave. As illustrated in FIG. 2, the time difference (T0-T1) between the R-wave in the EKG waveform at a time T0 and a peak m of the pulse wave at a time T1 can be expressed as t1. The time difference t1 is not the difference between the EKG waveform and the pulse wave. The "difference in phase PG" in FIG. 2 corresponds to the timing shift estimated by the estimating function 736. The difference in phase PG illustrated in FIG. 2 does not correspond to each peak of the pulse wave, but is estimated as one global parameter throughout the scan performed for the patient P. The estimated timing shift is referenced when the phase division is performed on the scan data.

Alternatively, the estimating function 736 may be configured to estimate a timing shift for each of the individual cycles in the pulse wave. In that situation, the estimated timing shifts may be used in correspondence with the individual cycles, in the reconstruction of the phase images. In yet another example, the estimating function 736 may be configured to estimate an average value of timing shifts estimated in correspondence with the individual cycles, as a global parameter for the timing shifts. Because the imaging period of the PET imaging process is long, the average value of the timing shifts is able to improve the level of precision of the timing shift estimation. Further, the estimated timing shift may be adjusted according to a user instruction received via the input interface 76.

As illustrated in FIG. 2, in the EKG-synchronized imaging process using the pulse wave, the interval between two R-waves adjacent to each other in the EKG waveform is divided into a plurality of phases. Although FIG. 2 illustrates an example in which the quantity of the plurality of phases resulting from the division is five (Ph1, Ph2, ... , and Ph5), possible embodiments are not limited to this example. In FIG. 2, according to the EKG waveform, the interval between the two pulse wave peaks adjacent to each other is divided into five phases (ph1', ph2', ... , and ph5'). In actual clinical examinations, the total quantity of the divided phases may be approximately sixteen in many situations. In this situation, the phase width may be fixed. For example, when an average pulse rate is 60 times per minute, an average cycle period is calculated as one second. In that situation, when the phase division yields five phases, the duration of one phase is 0.2 seconds. When the phase division yields sixteen phases, the duration of one phase is 0.0625 seconds. As illustrated in FIG. 2, when the scan data is reconstructed by referencing the phase division using the pulse wave, a reconstruction image in a phase (e.g., Ph1' in FIG. 2) not serving the purpose would be obtained. To perform the division process corresponding to the EKG waveform phases, the estimating function 736 is configured to calculate the difference in phase PG illustrated in FIG. 2.

For example, the estimating function 736 is configured to estimate the timing shift by performing a calculation based on a correspondence table (hereinafter, "physique difference correspondence table") indicating timing shifts with respect to physique information of the patient P. The physique difference correspondence table corresponds, for example, to a look-up table indicating the timing shifts corresponding to physique such as heights and weights of patients. The physique timing shift correspondence table is generated in advance and stored in the memory 75. More specifically, the estimating function 736 is configured to compare physique data such as the height and the weight of the patient P obtained from patient information of the patient P in an examination order output from a Radiology Information System (RIS) or a Hospital Information System (HIS), with the physique difference correspondence table. Subsequently, as a result of the comparison, the estimating function 736 is configured to identify a plurality of timing shifts close to the physique of the patient P. After that, the estimating function 736 is configured to estimate a timing shift by performing a calculation (e.g., calculating an average or a weighted average) on the identified plurality of timing shifts. In this situation, as a result of the comparison, when the physique difference correspondence table is found to have a timing shift that matches the physique of the patient P, the estimating function 736 is configured to identify the timing shift matching the physique of the patient P as a estimated timing shift. In that situation, the calculation is unnecessary. The estimating function 736 is configured to store the estimated timing shift into the memory 75.

In this situation, the timing shift estimating process performed by the estimating function 736 is not limited to the example using the physique difference correspondence table described above. For instance, the estimating function 736 may be configured to estimate the timing shift, by inputting the pulse wave information obtained by the pulse wave information obtaining function 735 to a trained model trained to output the timing shift in response to receiving an input of the pulse wave information of the patient P. The trained model is trained in advance and stored in the memory 75. The trained model is generated by training a pre-training model such as a Deep Neural Network (DNN), for example, while using a pulse wave and a timing shift related to each of a plurality of patients as learning data. In other examples, the learning data may be sets each made up of a pulse wave and an EKG waveform or may be sets each made up of a pulse wave and time data of R-wave peaks.

Further, as for the input to the trained model, geometric information related to the patient P may further be input. For example, when the camera 12 images the small changes in the complexion (or the neck part) of the patient P as the pulse wave information, the geometric information may be information indicating the distance between the face (or the neck part) of the patient P and the heart of the patient P. In that situation, the geometric information may be the distance itself or may be video data including the chest and the face (or the neck part) of the patient P imaged by the camera 12. Further, when an output of the pulse oximeter is obtained as the pulse wave information, the geometric information may be information indicating the distance between the pulse oximeter on the patient P and the heart of the patient P. In that situation, the geometric information may be the distance itself or may be video data including the chest of the patient P and the pulse oximeter imaged by the camera 12. In other examples, as the geometric information, it is possible to use any of various types of position determining images (which may be called scanogram images or scout images), an MR image, a simple X-ray image, or the like related to the patient P.

In this situation, the geometric information may be input according to a user instruction received via the input interface 76. A trained model to which the geometric information is further input is generated by training a pre-training model such as a Deep Neural Network (DNN), for example, while using a pulse wave, geometric information, and a timing shift related to each of a plurality of patients, as learning data. Because it is possible to apply known techniques to the pre-training model and to the learning method, explanations thereof will be omitted.

The display 74 is configured to display various types of information under control of the processing circuitry 73. For example, as the display 74, it is possible to use, as appropriate, a Cathode Ray Tube (CRT) display, a Liquid Crystal Display (LCD), an Organic Electroluminescence Display (OELD), a Light Emitting Diode (LED) display, a plasma display, or any of other arbitrary displays known in the relevant technical field. Further, the display 74 may be of a desktop type or may be configured by using a tablet terminal or the like capable of wirelessly communicating with the console 70.

The memory 75 is a storage device such as an HDD, an SSD, or an integrated circuit storage device configured to store therein various types of information. Alternatively, the memory 75 may be a Compact Disc Read-Only Memory (CD-ROM) drive, a Digital Versatile Disc (DVD) drive, or a drive device configured to read and write various types of information from and to a portable storage medium such as a flash memory. The memory 75 is configured to store therein, for example, various types of data related to the implementation of the data obtaining function 730, the reconstructing function 731, the image processing function 732, the imaging controlling function 733, the pulse wave information obtaining function 735, and the estimating function 736. The memory 75 is configured to store therein the scan data of the patient P obtained by scanning the patient P while employing the data obtaining function 730. The memory 75 is configured to store therein the pulse wave information obtained by the pulse wave information obtaining function 735. The memory 75 is configured to store therein the various types of programs related to the implementation of the data obtaining function 730, the reconstructing function 731, the image processing function 732, the imaging controlling function 733, the pulse wave information obtaining function 735, and the estimating function 736. In addition, the memory 75 is configured to store therein the physique difference correspondence table, the trained model, the geometric information, and the like used in the implementation of the estimating function 736.

The input interface 76 is configured to receive various types of input operations from the user, to convert the received input operations into electrical signals, and to output the electrical signals to the processing circuitry 73. For example, as the input interface 76, it is possible to use a mouse, a keyboard, a trackball, a switch, a button, a joystick, a touchpad, a touch panel display, and/or the like, as appropriate, for instance. In the present embodiment, the input interface 76 does not necessarily have to include physical operational component parts such as the mouse, the keyboard, the trackball, the switch, the button, the joystick, the touchpad, the touch panel display, and/or the like. For instance, possible examples of the input interface 76 include electrical signal processing circuitry configured to receive an electrical signal corresponding to an input operation from an external input apparatus provided separately from the apparatus and to output the electrical signal to the processing circuitry 73. Alternatively, the input interface 76 may be configured by using a tablet terminal or the like capable of wirelessly communicating with the console 70.

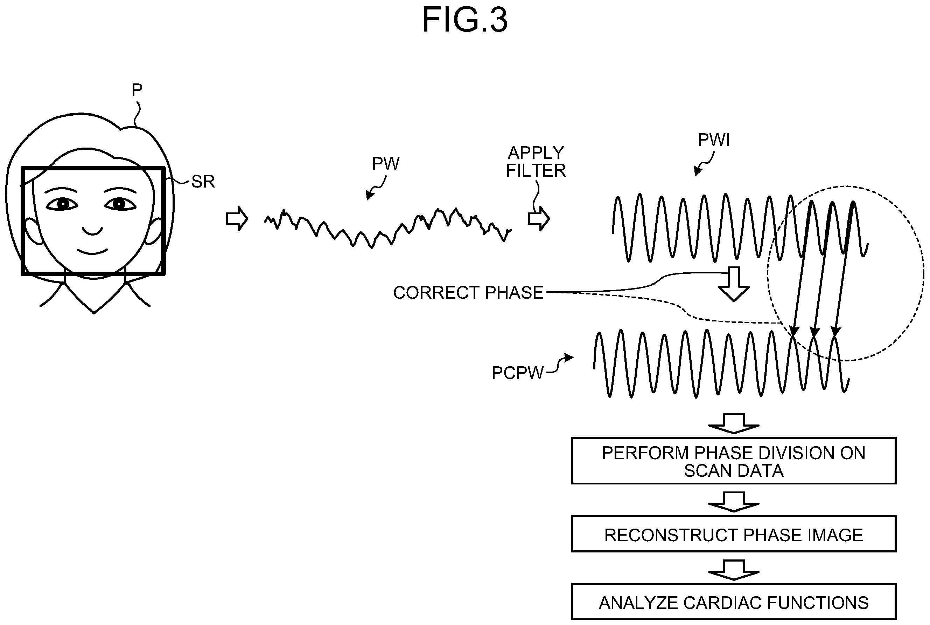

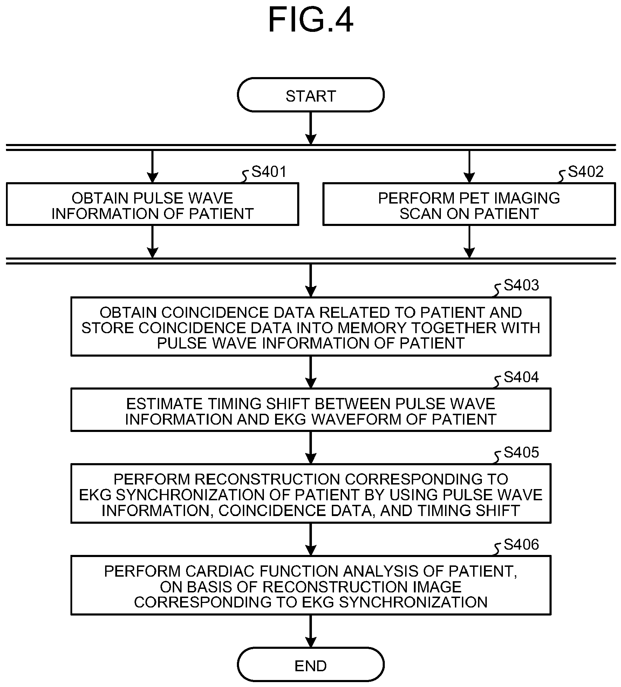

An overall configuration of the PET-CT apparatus 1 has thus been explained. Next, a procedure in the EKG-synchronized reconstruction process will be explained, with reference to FIGS. 3 and 4. Further, to explain a specific example, it is assumed that the PET gantry 10 is provided with the camera 12. In addition, it is assumed that the scan data subject to the EKG-synchronized reconstruction process is coincidence event data.

FIG. 3 is a drawing illustrating an example of an outline of the EKG-synchronized reconstruction process. As illustrated in FIG. 3, a pulse wave PW is obtained on the basis of small changes in the face complexion of the patient P in an imaged range SR of the camera 12. In this situation, as the scan data, the coincidence event data is obtained, while being kept in correspondence with the pulse wave PW. Pulse wave information PWI is obtained by applying a filter for noise elimination or the like to the pulse wave PW. Alternatively, when a timing shift PG is estimated by using a trained model, a pulse wave PW may be obtained as the pulse wave information.

When the timing shift PG is estimated as illustrated in the dotted-line enclosure in FIG. 3, a phase correction is performed on the pulse wave information PWI by making a shift with the timing shift. For example, the phase correction is performed by the reconstructing function 731 in the EKG-synchronized reconstruction process. Subsequently, as illustrated in FIG. 3, the reconstructing function 731 is configured to perform the phase division on the coincidence event data, by using phase-corrected pulse wave information PCPW. Accordingly, the coincidence event data is brought into correspondence with each of the plurality of phases. Subsequently, as illustrated in FIG. 3, the reconstructing function 731 is configured to reconstruct a phase image on the basis of the coincidence event data resulting from the phase division. The reconstructed phase image will be used, for example, for a cardiac function analysis by the image processing function 732, as illustrated in FIG. 3.

FIG. 4 is a flowchart illustrating an example of a procedure in the EKG-synchronized reconstruction process.

### EKG-synchronized Reconstruction Process

### Step S401:

By employing the pulse wave information obtaining function 735, the processing circuitry 73 obtains pulse wave information of the patient P, starting before a PET imaging process is performed on the patient P. The pulse wave information obtaining function 735 stores the obtained pulse wave information into the memory 75.

### Step S402:

By employing the imaging controlling function 733, the processing circuitry 73 performs a scan on the patient P by performing a PET imaging process. In addition, at a stage prior to the present step, the imaging controlling function 733 performed a CT imaging process on the patient P, to obtain attenuation data used for reconstructing coincidence event data.

### Step S403:

By employing the data obtaining function 730, the processing circuitry 73 obtains the CT raw data and the coincidence event data. The data obtaining function 730 stores the obtained data into the memory 75, so as to be kept in correspondence with the pulse wave information.

### Step S404:

By employing the estimating function 736, the processing circuitry 73 estimates a timing shift between the pulse wave information and the EKG waveform of the patient P. The estimating function 736 stores the estimated timing shift into the memory 75.

### Step S405:

By employing the reconstructing function 731, the processing circuitry 73 carries out image reconstruction corresponding to EKG synchronization of the patient P, by using the pulse wave information, the coincidence data, and the timing shift. More specifically, the reconstructing function 731 identifies times corresponding to the R-wave (hereinafter, "R-wave times"), by applying the timing shift to the pulse wave information. Subsequently, on the basis of the R-wave times, the reconstructing function 731 performs the phase division on the pulse wave information. Among the plurality of phases resulting from the phase division, the reconstructing function 731 reconstructs a PET image corresponding to the EKG synchronization, by using the coincidence data and attenuation data included in a phase width related to the R-wave times. Because it is possible to use any of known methods for the image reconstruction using the coincidence data and the attenuation data, explanations thereof will be omitted.

### Step S406:

By employing the image processing function 732, the processing circuitry 73 performs a cardiac function analysis on the PET image corresponding to the EKG synchronization. Because it is possible to use any of known methods for the cardiac function analysis performed on the PET image, explanations thereof will be omitted. Thus, the image processing function 732 has obtained a result similar to a result of a cardiac function analysis based on EKG-synchronized imaging process. The image processing function 732 stores the result of the cardiac function analysis into the memory 75.

The medical image diagnosis apparatus 1 according to the first embodiment described above is configured: to obtain the scan data generated by scanning the patient P; to obtain the pulse wave information of the patient P along with the scan; and to perform the image reconstruction corresponding to the EKG synchronization of the patient P, by using the pulse wave information and the scan data. More specifically, the medical image diagnosis apparatus 1 according to the first embodiment is configured to estimate the timing shift between the pulse wave information and the EKG waveform of the patient P and to perform the image reconstruction corresponding to the EKG synchronization by further using the estimated timing shift. For example, the medical image diagnosis apparatus 1 according to the first embodiment is configured to estimate the timing shift by performing the calculation based on the correspondence table indicating the timing shift in relation to the physique information of the patient P. Further, the medical image diagnosis apparatus 1 according to the first embodiment may be configured to estimate the timing shift by inputting the obtained pulse wave information to the trained model trained to output the timing shift in response to receiving the input of the pulse wave information. Further, the medical image diagnosis apparatus 1 according to the first embodiment may be configured to estimate the timing shift by inputting the obtained pulse wave information and the physique information of the patient P, to the trained model trained to output the timing shift in response to receiving the input of the pulse wave information and the geometric information related to the patient P.

Consequently, the medical image diagnosis apparatus 1 according to the first embodiment makes it possible to perform the imaging process, without the need to attach the electrocardiographic ports to the patient P and to route the cables around for the purpose of obtaining the EKG waveform of the patient P. It is therefore possible, by using the recorded pulse wave, to carry out the heartbeat-synchronized reconstruction (the EKG-synchronized image reconstruction) equivalent to EKG synchronization with a CT image, a PET image, a SPECT image, an MR image or the like. In other words, the present medical image diagnosis apparatus 1 is capable of performing the image reconstruction corresponding to the EKG synchronization of the patient P by using the pulse wave information, without the need to use the electrocardiographic ports and the cables which may be a cause of image quality degradation in the reconstruction image and without the need to obtain EKG waveforms. As a result, the present medical image diagnosis apparatus 1 is able to improve the image quality compared to that of reconstruction images based on normal EKG synchronization and thus makes it possible to perform the cardiac function analysis with an excellent level of precision. Consequently, the present medical image diagnosis apparatus 1 is able to improve the level of precision of the medical examination performed for the patient P.

Further, the present medical image diagnosis apparatus 1 is able to alleviate the trouble of attaching various types of apparatuss related to a plurality of displacement monitors to the patient P, for so-called double-gate imaging combined with respiratory synchronization. Consequently, the present medical image diagnosis apparatus 1 is able to enhance operability in the EKG-synchronized examination performed for the patient P and to thus improve efficiency of the medical examination (a throughput of the medical examination) related to the EKG synchronization.

### Modification Example

In the present modification example, the interval between two pulse wave peaks adjacent to each other in the pulse wave information is divided into a plurality of phases, so as to generate a time-volume curve related to the heart of the patient P on the basis of a plurality of reconstruction images generated in correspondence with the plurality of phases and to further estimate a timing shift on the basis of the generated time-volume curve.

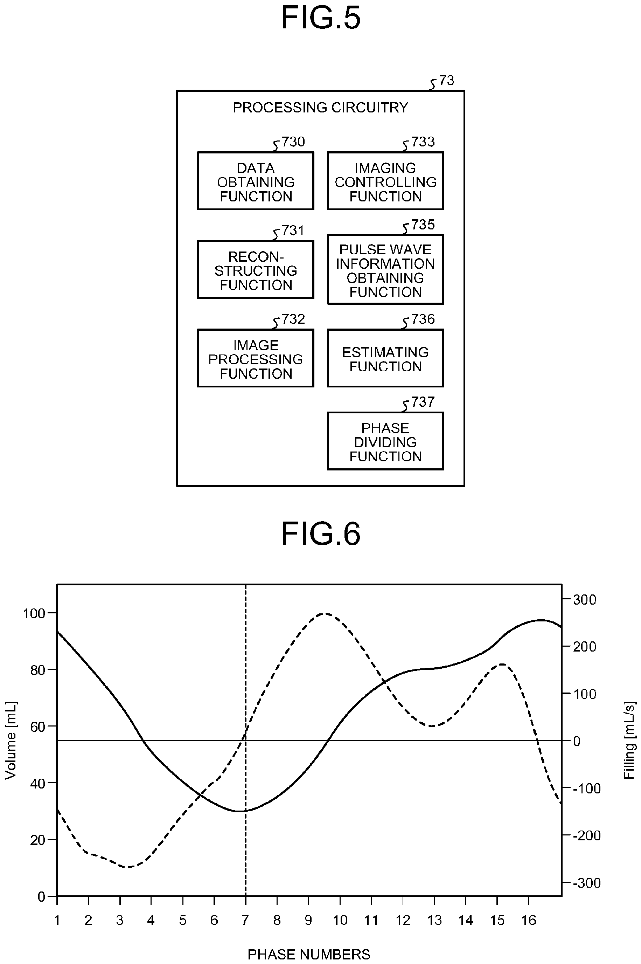

FIG. 5 is a drawing illustrating an example of a functional configuration of the processing circuitry 73 according to the present modification example. As illustrated in FIG. 5, the processing circuitry 73 according to the present modification example further includes a phase dividing function 737. The processing circuitry 73 realizing the phase dividing function 737 corresponds to a phase dividing unit. By employing the phase dividing function 737, the processing circuitry 73 is configured, in the pulse wave information obtained by the pulse wave information obtaining function 735, to divide the interval between two pulse wave peaks adjacent to each other into a plurality of phases.

In the following sections, to explain a specific example, it will be assumed that the quantity of the plurality of phases (i.e., the division number) is sixteen, for example. However, the division number is not limited to sixteen and may arbitrarily be set (e.g., five, as illustrated in FIG. 2).

By employing the reconstructing function 731, the processing circuitry 73 is configured to generate a plurality of reconstruction images corresponding to the plurality of phases, on the basis of the scan data included in each of the plurality of phases. For example, the reconstructing function 731 is configured to generate a plurality of PET images respectively corresponding to the plurality of phases, on the basis of the coincidence event data included in each of the plurality of phases.

By employing the estimating function 736, the processing circuitry 73 is configured to generate a time-volume curve related to the heart of the patient P, on the basis of the plurality of reconstruction images. For example, the time-volume curve is a curve indicating changes in the course of time or the like, regarding the volume of the ventricles of the heart of the patient P. Alternatively, the process of generating the time-volume curve may be realized by the image processing function 732. The estimating function 736 is configured to estimate a timing shift on the basis of the time-volume curve. For example, the estimating function 736 is configured to identify a phase in which the volume is minimum from the time-volume curve. Subsequently, the estimating function 736 is configured to determine, with respect to a phase having a pulse wave peak, the time period between the earlier phase and the identified phase as the timing shift. Further, the phase dividing function 737 may perform phase division again, by using the determined timing shift. In this situation, the reconstructing function 731 may generate a sharp reconstruction image with respect to each of the divided phases, by using the re-divided phases.

FIG. 6 is a chart illustrating an example of the time-volume curve. As illustrated in FIG. 6, the phase having a minimum volume is the seventh phase. In this situation, the estimating function 736 is configured to identify the time difference between the first phase and the seventh phase as the timing shift.

The medical image diagnosis apparatus 1 according to the modification example of the first embodiment described above is configured: to divide the interval between the two pulse wave peaks adjacent to each other in the pulse wave information into the plurality of phases; to generate the plurality of reconstruction images corresponding to the plurality of phases on the basis of the scan data included in each of the plurality of phases; to generate the time-volume curve related to the heart of the patient P, on the basis of the plurality of reconstruction images; and to estimate the timing shift on the basis of the time-volume curve. Because advantageous effects of the present modification example are the same as those of the first embodiment, explanations thereof will be omitted.

### Second Embodiment

In the present embodiment, the image reconstruction corresponding to the EKG synchronization is carried out without estimating the timing shift. The processing circuitry 73 according to the present embodiment includes the plurality of functions obtained by excluding the estimating function 736 from the plurality of functions illustrated in FIG. 5.

By employing the phase dividing function 737, the processing circuitry 73 is configured to divide the interval between two pulse wave peaks adjacent to each other in the pulse wave information, into a plurality of phases. The processing circuitry 73 realizing the phase dividing function 737 corresponds to a phase dividing unit. In the following sections, to explain a specific example, it will be assumed that the quantity of the plurality of phases (i.e., the division number) is seven, for example. However, the division number is not limited to seven and may arbitrarily be set (e.g., five, as illustrated in FIG. 2).

By employing the reconstructing function 731, the processing circuitry 73 is configured to shift an initial phase corresponding to one of the peaks among the plurality of phases divided by the phase dividing function 737, multiple times toward the past, once per predetermined time interval. The predetermined time interval is one of partial phase widths obtained by equally dividing the phase width. The predetermined time interval is set in advance, for example, and stored in the memory 75. The predetermined time interval may be, for example, one-fifth or one-tenth of the phase width. In the following sections, to explain a specific example, it will be assumed that the predetermined time interval is one-fifth of the phase width. For example when the time period between the two pulse wave peaks adjacent to each other in the pulse wave information is one second, the phase width of each of the plurality of phases is one-seventh of a second. In that situation, the predetermined time interval is 1/35 seconds. Further, to explain a specific example, it will be assumed that the initial phase is a phase corresponding to the earlier peak between the two pulse wave peaks among the plurality of phases.

By employing the reconstructing function 731, the processing circuitry 73 is configured to generate a plurality of reconstruction images corresponding to the number of times the shift was made, on the basis of the scan data included in the two or more initial phases that were shifted. In the above example, by using the scan data included in each of five initial phases respectively corresponding to the shift made five times, the reconstructing function 731 is configured to generate five reconstruction images corresponding to the five initial phases.

By employing the reconstructing function 731, the processing circuitry 73 is configured to identify the sharpest image among the plurality of reconstruction images, as a reconstruction image corresponding to the EKG synchronization. For example, the reconstructing function 731 is configured to calculate a sharpness index with respect to each of the plurality of reconstruction images. With respect to each of the plurality of reconstruction images, the sharpness index is defined as the sum of squares of partial derivative values, in the x direction and the y direction (i.e., the height and the width directions) of the pixel values in the entire image. The sharpness index is an index indicating the degree of being out of focus of each of the plurality of reconstruction images.

Possible examples of the sharpness index are not limited to the calculation example described above. As long as the index is able to quantify the extent of being out of focus of the images, it is possible to use any of known methods, as appropriate. The reconstructing function 731 is configured to identify a reconstruction image having the smallest sharpness index, i.e., the reconstruction being out of focus with the lowest degree, as the reconstruction image corresponding to the EKG synchronization. Alternatively, the process of identifying the reconstruction image corresponding to the EKG synchronization may be performed by the image processing function 732 or the like.

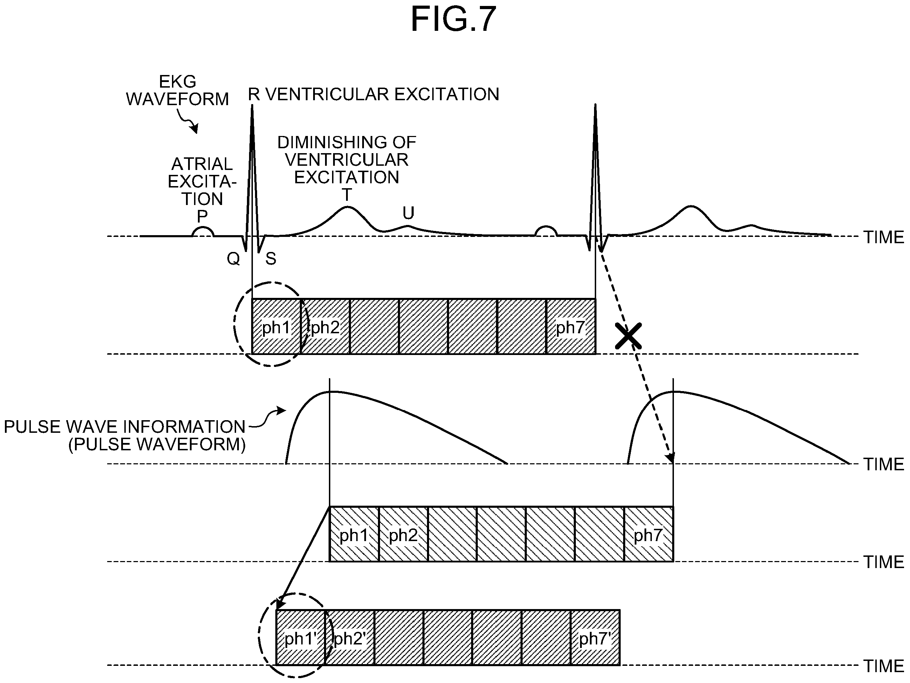

FIG. 7 is a drawing illustrating the phase division in the pulse wave information (the pulse waveform) and an example of a phase ph1' of the reconstruction image corresponding to the EKG synchronization based on shifting of an initial phase ph1 in the pulse waveform. The phase ph1 in the EKG waveform corresponds to an excitation period of ventricular muscles. For this reason, in the present embodiment, because the cycle of the heartbeats is not constant but is variable, attention is paid to the phase ph1' in which the sharpest reconstruction image is obtained. Further, in the present embodiment also, the difference between the EKG waveform and the pulse waveform is assumed to be global and constant. As illustrated in FIG. 7, on the basis of the pulse wave peaks adjacent to each other, the pulse waveform is divided by the phase dividing function 737 into seven phases (ph1, ph2, ... , and ph7). The reconstructing function 731 is configured to generate reconstruction images by shifting the initial phase ph1 toward the past, once every predetermined time interval Δt used as a trial and to further identify one of the reconstruction images corresponding to the EKG synchronization.

In FIG. 7, ph1' is the phase corresponding to the reconstruction image that corresponds to the EKG synchronization. In this situation, on the basis of the scan data corresponding to each of the other plurality of shifted phases (ph2', ... and ph7'), the reconstructing function 731 may generate a plurality of reconstruction images corresponding to the other plurality of shifted phases (ph2', ... , and ph7'). The plurality of reconstruction images corresponding to the plurality of shifted phases (ph1', ph2', ... , and ph7') may be used by the image processing function 732 for a cardiac function analysis, for example.

The medical image diagnosis apparatus 1 according to the second embodiment described above is configured to divide the interval between the two pulse wave peaks adjacent to each other in the pulse wave information into the plurality of phases; to shift the initial phase corresponding to at least one of the peaks among the plurality of phases, multiple times toward the past at the predetermined time intervals; and to generate the plurality of reconstruction images corresponding to how many times the shift was made, on the basis of the scan data included in the two or more initial phases that were shifted. Further, the medical image diagnosis apparatus 1 according to the second embodiment is configured to identify the sharpest image among the plurality of reconstruction images as the reconstruction image corresponding to the EKG synchronization. Because advantageous effects of the present embodiment are the same as those of the first embodiment, explanations thereof will be omitted.

### Application Examples

In the present application example, the sharpest image is identified by inputting the plurality of reconstruction images to a trained model trained to identify the sharpest image among the plurality of reconstruction images in response to receiving an input of the plurality of reconstruction images generated in the second embodiment. In this situation, the sharpest image corresponds to an image close to a typical phase image immediately after the R-wave. By employing the reconstructing function 731, the processing circuitry 73 is configured to identify the sharpest image by inputting the plurality of reconstruction images to the trained model trained to identify the sharpest image among the plurality of reconstruction images in response to receiving the input of the plurality of reconstruction images.

The trained model is trained in advance and is stored in the memory 75. The trained model is generated by training a pre-training model such as a Deep Neural Network (DNN), for example, while using a plurality of reconstruction images and EKG-synchronized reconstruction images related to each of a plurality of patients, as learning data. Because it is possible to apply known techniques to the pre-training model and to the learning method, explanations thereof will be omitted.

The medical image diagnosis apparatus 1 according to the present application example is configured to divide the interval between the two pulse wave peaks adjacent to each other in the pulse wave information into the plurality of phases; to shift the initial phase corresponding to the one of the peaks among the plurality of phases, multiple times toward the past at the predetermined time intervals; to generate the plurality of reconstruction images corresponding to how many times the shift was made, on the basis of the scan data included in the two or more initial phases that were shifted; and to identify the sharpest image by inputting the plurality of reconstruction images to the trained model trained to identify the sharpest image among the plurality of reconstruction images in response to receiving the input of the plurality of reconstruction images. Because advantageous effects of the present application example are the same as those of the first embodiment, explanations thereof will be omitted.

When technical concept of the present embodiment is realized as an image reconstruction method, the image reconstruction method includes: obtaining scan data by scanning the patient P; obtaining pulse wave information of the patient P, along with the scan; and performing image reconstruction corresponding to EKG synchronization of the patient P, by using the pulse wave information and the scan data. Because a processing procedure and advantageous effects of the present image reconstruction method are the same as those of the first embodiment, explanations thereof will be omitted.

When technical concept of the present embodiment is realized as an image reconstruction program, the image reconstruction program causes a computer to realize: obtaining scan data by scanning the patient P; obtaining pulse wave information of the patient P, along with the scan; and performing image reconstruction corresponding to EKG synchronization of the patient P, by using the pulse wave information and the scan data. In this situation, the program capable of causing the computer to implement the method may be distributed as being stored in a storage medium such as a magnetic disk (e.g., a hard disk), an optical disk (e.g., a CD-ROM or a DVD), or a semiconductor memory. Because a processing procedure and advantageous effects of the present image reconstruction program are the same as those of the first embodiment, explanations thereof will be omitted.

In an embodiment, the pulse wave is a detectable signal which varies with the heartbeat of the examined subject. The pulse wave may be obtained from variations in the appearance of an area being imaged or variations in a physiological measurement, e.g. pulse wave oximetry.

According to at least one aspect of the embodiments and the like described above, it is possible to perform the image reconstruction corresponding to the EKG synchronization, without the need to attach electrocardiographic ports to the patient P and to route cables around.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims.

## Claims

1. A medical image diagnosis apparatus (1) comprising:
a data obtaining unit (730) configured to obtain scan data generated by scanning an examined subject;
a pulse wave information obtaining unit (735) configured to obtain pulse wave information of the examined subject, along with the scan; and
a reconstructing unit (731) configured to perform image reconstruction corresponding to electrocardiogram synchronization of the examined subject, by using the pulse wave information and the scan data.

2. The medical image diagnosis apparatus (1) according to claim 1, further comprising an estimating unit (736) configured to estimate a timing shift between the pulse wave information and an electrocardiogram waveform of the examined subject, wherein
the reconstructing unit (731) is configured to perform the image reconstruction corresponding to the electrocardiogram synchronization, by further using the timing shift.

3. The medical image diagnosis apparatus (1) according to claim 2, wherein the estimating unit (736) is configured to estimate the timing shift, through a calculation based on a correspondence table indicating timing shifts corresponding to physique information of the examined subject.

4. The medical image diagnosis apparatus (1) according to claim 2, wherein the estimating unit (736) is configured to estimate the timing shift, by inputting the pulse wave information to a trained model trained to output the timing shift in response to receiving an input of the pulse wave information.

5. The medical image diagnosis apparatus (1) according to claim 2, wherein the estimating unit (736) is configured to estimate the timing shift, by inputting the pulse wave information and physique information of the examined subject to a trained model trained to output the timing shift, in response to receiving an input of the pulse wave information and geometric information related to the examined subject.

6. The medical image diagnosis apparatus (1) according to claim 2, further comprising a phase dividing unit (737) configured to divide an interval between two pulse wave peaks adjacent to each other in the pulse wave information, into a plurality of phases, wherein
the reconstructing unit (731) is configured to generate a plurality of reconstruction images corresponding to the plurality of phases, on a basis of the scan data included in each of the plurality of phases, and
the estimating unit (736) is configured
to generate a time-volume curve related to a heart of the examined subject on a basis of the plurality of reconstruction images; and
to estimate the timing shift on a basis of the time-volume curve.

7. The medical image diagnosis apparatus (1) according to any one of claims 1 to 6, wherein the pulse wave information obtaining unit (735) is configured either to contactlessly obtain the pulse wave information from the examined subject or to obtain the pulse wave information from a pulse wave meter provided in an end part of the examined subject within a non-imaged range during the scan.

8. The medical image diagnosis apparatus (1) according to claim 1, further comprising a phase dividing unit (737) configured to divide an interval between two pulse wave peaks adjacent to each other in the pulse wave information, into a plurality of phases, wherein
the reconstructing unit (731) is configured
to shift an initial phase corresponding to one of the peaks among the plurality of phases, multiple times toward past at predetermined time intervals; and
to generate a plurality of reconstruction images corresponding to how many times the shift was made, on a basis of the scan data included in two or more of the initial phases that were shifted.

9. The medical image diagnosis apparatus according to claim 8, wherein the processing circuitry is configured to identify a sharpest image among the plurality of reconstruction images as a reconstruction image corresponding to the electrocardiogram synchronization.

10. The medical image diagnosis apparatus (1) according to claim 1, further comprising a phase dividing unit (737) configured to divide an interval between two pulse wave peaks adjacent to each other in the pulse wave information, into a plurality of phases, wherein
the reconstructing unit (731) is configured
to shift an initial phase corresponding to one of the peaks among the plurality of phases, multiple times toward past at predetermined time intervals;
to generate a plurality of reconstruction images corresponding to how many times the shift was made, on a basis of the scan data included in two or more of the initial phases that were shifted; and
to identify a sharpest image among the plurality of reconstruction images, by inputting the plurality of reconstruction images to a trained model trained to identify the sharpest image in response to receiving an input of the plurality of reconstruction images.

11. An image reconstruction method comprising:
obtaining scan data by scanning an examined subject;
obtaining pulse wave information of the examined subject, along with the scan; and
performing image reconstruction corresponding to electrocardiogram synchronization of the examined subject, by using the pulse wave information and the scan data.

12. A computer-readable storage medium storing therein an image reconstruction program that causes a computer to realize:
obtaining scan data by scanning an examined subject;
obtaining pulse wave information of the examined subject, along with the scan; and
performing image reconstruction corresponding to electrocardiogram synchronization of the examined subject, by using the pulse wave information and the scan data.
